# EUROPEAN PATENT APPLICATION

(11) **EP 4 810 084 A1**
(43) Date of publication of application: **23.09.2026**
(21) Application number: 24891290.9
(22) Date of filing: 06.11.2024
(51) Int. Cl.: A61M 5/30

(54) **INJECTOR AND METHOD FOR INJECTING SOLUTION INTO INTENDED SITE USING INJECTOR**

(30) Priority: 14.11.2023 JP 2023193370
(71) Applicant: Daicel Corporation, Osaka-shi, Osaka 530-0011 (JP)
(72) Inventor: TERAI, Kazuhiro, Tokyo 108-8230 (JP)
(74) Representative: Grünecker Patent- und Rechtsanwälte PartG mbB
(86) International application number: PCT/JP2024/039386
(87) International publication number: WO 2025/105251

(57) **Abstract**

An object is to at least provide an injector having a fragile tissue as an injection target. The problems are solved by an injector for injecting a solution containing a biofunctional substance into a target, the injector including: an accommodation part configured to accommodate the solution; a nozzle part configured to communicate with the accommodation part, the nozzle part having an ejection port for ejecting the solution toward the target; and a pressurization part configured to eject the solution from the ejection port toward the target by pressurizing the solution accommodated in the accommodation part when actuated, in which a value in which a kinetic energy of the solution to be ejected is divided by an area of the ejection port is 40 mJ/mm² or more and 1500 mJ/mm² or less.

## Description

### Technical Field

The present disclosure relates to an injector and a method for injecting a solution into an intended site using the injector.

### Background Art

Injectors for injecting medical liquid into a target include a needle injector that performs injection with an injection needle and a needleless injector that performs injection without an injection needle. A catheter including an injection needle and a drive source for transporting medical liquid to the target, a porous injection needle, and the like are also utilized.

Among these, a needleless injector may be configured to eject an injection component by applying pressure to an accommodating chamber in which an injection solution is accommodated by a pressurized gas, a spring, or an electromagnetic force. For example, a configuration is adopted in which a plurality of nozzle holes are formed inside an injector body, and pistons that are driven at the time of ejection are disposed in correspondence with the respective nozzle holes (Patent Document 1). With this configuration, uniform injection into the target is intended to be achieved by jetting an injection solution from the plurality of nozzle holes at the same time. Then, a plasmid containing a luciferase gene is injected into a rat, which allows cell transfer with high efficiency.

There is a form in which a pressurized gas is utilized as an ejection power source of an injection solution in a needleless injector. For example, a pressurization form in which high pressurization is instantaneously performed in the initial stage of ejection and then the pressurizing force is gradually reduced over 40 to 50 msec is exemplified (Patent Document 2).

In a mode in which jet injection is performed as described above, subcutaneous and intradermal injection targets are mainly investigated (Patent Document 1).

### Citation List

### Patent Literature

Patent Document 1: JP 2004-358234 A
Patent Document 2: US 2005/0010168 A

### Summary of Invention

### Technical Problem

The present inventor has investigated performing jet injection into a fragile tissue such as a liver or a spleen as an injection target, and has found that a solution may penetrate the target when the solution is injected by pressing a needleless injector against the target.

An object of the present disclosure is at least as follows. That is, an injector having a fragile tissue as an injection target is provided. A method for injecting a solution into a target using the injector is provided.

### Solution to Problem

As a result of intensive studies to solve the above problems, the present inventor has found that the above problems can be solved by using a predetermined injector.

That is, an overview of the present disclosure is as described below.
[1] An injector for injecting a solution containing a biofunctional substance into a target, the injector including:
   an accommodation part configured to accommodate the solution;
   a nozzle part configured to communicate with the accommodation part, the nozzle part having an ejection port for ejecting the solution toward the target; and
   a pressurization part configured to eject the solution from the ejection port toward the target by pressurizing the solution accommodated in the accommodation part when actuated, in which
   a value in which a kinetic energy of the solution to be ejected is divided by an area of the ejection port is 40 mJ/mm² or more and 1500 mJ/mm² or less.
[2] The injector according to [1], in which a fluid volume of the solution is 0.3 µL or more and 4.0 µL or less.
[3] The injector according to [1] or [2], in which an area of the ejection port is 0.001 mm² or more and 1.25 mm² or less.
[4] The injector according to any of [1] to [3], in which an area of the ejection port is 0.001 mm² or more and 0.02 mm² or less.
[5] The injector according to any of [1] to [4], in which the biofunctional substance is one type or more selected from the group consisting of a nucleic acid, a peptide, a protein, a sugar, and a low-molecular-weight compound, as well as a complex thereof.
[6] The injector according to any of [1] to [5], in which
   the target is one or more selected from the group consisting of fat, a skeletal muscle, a ligament, an aorta, a superior vena cava, a brain, an eyeball, an eardrum, an inner ear, a nerve, a trachea, a bronchus, a lung, an esophagus, a stomach, a liver, a kidney, an adrenal gland, a spleen, a gallbladder, a pancreas, a large intestine, a small intestine, a duodenum, a cecum, an appendix, a rectum, a greater omentum, a ureter, bone marrow, a lymph node, a lymphatic network, a thyroid gland, a sebaceous gland, a sweat gland, a salivary gland, a thymus, a mammary gland, a prostate, a fallopian tube, an ovary, a uterus, a cervix, a testicle, a vas deferens, and a seminal vesicle, as well as
   a mucous membrane and a connective tissue associated with any one of these, as well as
   a neoplasm occurring in any one of these.
[7] A method for injecting the solution containing the biofunctional substance into the target using the injector according to any of [1] to [6].

### Advantageous Effects of Invention

The present disclosure can at least provide an injector having a fragile tissue as an injection target. The present disclosure can provide a method for injecting a solution into a target using the injector.

### Brief Description of Drawings

FIG. 1 is an overall view of an injector according to an embodiment of the present disclosure.
FIG. 2 is a cross-sectional view illustrating the vicinity of a tip end portion of the injector illustrated in FIG. 1.
FIG. 3 is an overall view of an injector according to an embodiment of the present disclosure.
FIG. 4 is a graph showing the luminescence amount (RFU/µL) in a case in which pLuc of each fluid volume is introduced into a mouse liver or spleen. n = 4, means ± SD.

### Description of Embodiments

Each of the configurations, combinations thereof, and the like in each of the embodiments is an example, and additions, omissions, substitutions, and other changes of the configurations may be made as appropriate without departing from the spirit of the present disclosure. The present disclosure is not limited by the embodiments and is limited only by the claims. Each embodiment disclosed in the present specification can be combined with any other feature disclosed herein.

Further, a numerical range expressed by "to" implies a range including the numerical values described before and after "to" as the lower limit value and the upper limit value. Specifically, "A to B" implies a value that is A or more and B or less.

An embodiment of the present disclosure is
an injector for injecting a solution containing a biofunctional substance into a target, the injector including:
an accommodation part configured to accommodate the solution;
a nozzle part configured to communicate with the accommodation part, the nozzle part having an ejection port for ejecting the solution toward the target; and
a pressurization part configured to eject the solution from the ejection port toward the target by pressurizing the solution accommodated in the accommodation part when actuated, in which
a value in which a kinetic energy of the solution to be ejected is divided by an area of the ejection port is 40 mJ/mm² or more and 1500 mJ/mm² or less.

### Solution Containing Biofunctional Substance

The solution that can be injected by the injector according to the present embodiment contains a biofunctional substance. The biofunctional substance is not particularly limited as long as the substance exhibits physiological activity in the injection target. The biofunctional substance may be one type or may be a plurality of types. The biofunctional substance may be a natural product or may be an artificially synthesized product.

The biofunctional substance is preferably one type or more selected from the group consisting of a nucleic acid, a peptide, a protein, a sugar, and a low-molecular-weight compound, as well as a complex thereof.

Examples of the nucleic acid include deoxyribonucleic acid (DNA), ribonucleic acid (RNA), and peptide nucleic acid (PNA). The nucleic acid may be a nucleic acid including a portion encoding a protein, or a nucleic acid not including a portion encoding a protein (non-coding nucleic acid).

Examples of the peptides and the proteins include antigens (that is, those that produce antibodies against the peptides and proteins), antibodies, peptide vaccines, protein vaccines, peptide hormones, protein hormones, growth factors, cytokines, blood coagulation factors, serum albumin, digestive enzymes, anti-inflammatory peptides, and anti-inflammatory proteins.

The low-molecular-weight compound usually refers to a compound having a molecular weight of 2000 or less, but is not limited to this, and includes a compound that can be treated as a low-molecular-weight compound in the art. Examples of a preferable range of the molecular weight of the low-molecular-weight compound include 50 or more, or 100 or more. Further examples include 2000 or less and 1000 or less. That is, examples include 50 to 2000, 50 to 1000, and 100 to 2000.

The complex refers to a substance in which two or more substances corresponding to any of a nucleic acid, a peptide, a protein, a sugar, or a low-molecular-weight compound is integrated by a covalent bond, an ionic bond, a hydrogen bond, a hydrophobic interaction, or the like.

In the present disclosure, physiological activity refers to an effect on a specific physiological regulatory function of a living body. An evaluation index of the physiological activity can be set as appropriate according to purpose, and may be either a qualitative index or a quantitative index, but a quantitative index is preferred. For example, a specific mRNA amount, protein amount, cytokine amount, antibody titer, or number of cells of a specific cell type can be used as an index. These quantitative indices can be quantified by methods known in the art.

In particular, when the biofunctional substance is a nucleic acid containing a portion encoding a protein, an amount of the protein encoded by the nucleic acid can be quantified and used as an index for evaluating the physiological activity. Further, the activity of the protein may be quantitatively evaluated. For example, in a case in which the protein is luciferase, the physiological activity can be evaluated by measuring the intensity of bioluminescence.

In a case in which the biofunctional substance is a nucleic acid, the nucleic acid may be incorporated into a viral vector or carried by a lipid nanoparticle and contained in the solution, but the viral vector or the lipid nanoparticle need not be used. When the viral vector or the lipid nanoparticle is not used, the possibility that a side reaction such as anaphylaxis is induced in the target can be reduced.

The content of the biofunctional substance with respect to the total amount of the solution can be appropriately set based on the type of biofunctional substance, the physiological activity exhibited by the biofunctional substance in the target into which the biofunctional substance is injected, or the like. Examples thereof include 0.0001 mg/mL or more, 0.001 mg/mL or more, and 0.01 mg/mL or more. Examples thereof include 1000 mg/mL or less and 100 mg/mL or less. That is, examples include 0.0001 to 1000 mg/mL, 0.001 to 100 mg/mL, and 0.01 to 100 mg/mL.

In addition to the biofunctional substance, the solution may contain common additives such as a buffering agent, a tonicity agent, a pH adjusting agent, an antioxidant, a thickener, a stabilizer, a wetting agent, an emulsifier, and a binder, as necessary.

As the buffering agent, any of various types can be used, for example, phosphate-based buffer solutions (for example, phosphate buffer, phosphate buffered saline (PBS) (which may be PBS(+) or PBS(-)), Dulbecco's phosphate buffered saline (D-PBS), citrate-phosphate buffer, citrate-phosphate buffered saline), citrate buffering agents, tris(hydroxymethyl)aminomethane-HCl buffering agents (Tris-HCl buffering agent), acetate buffering agents, Good's buffering agents (for example, HEPES-NaOH buffering agent), amino acid-based buffering agents (for example, glycine-HCl buffering agent, glycine-NaOH buffering agent, glycylglycine-NaOH buffering agent, glycylglycine-KOH buffering agent), and imidazole buffering agents.

Among these, a buffer solution that uses phosphoric acid is preferable from the viewpoint of versatility.

Examples of the tonicity agent include an ionic tonicity agent and a nonionic tonicity agent.

Examples of the ionic tonicity agent include salts such as sodium chloride, potassium chloride, calcium chloride, and magnesium chloride.

Examples of the nonionic tonicity agent include glycerin, propylene glycol, polyethylene glycol, glucose, sorbitol, mannitol, trehalose, maltose, and sucrose.

Among these, sodium chloride is preferable from the viewpoint of versatility.

Examples of the pH adjusting agent include hydrochloric acid, phosphoric acid, citric acid, acetic acid, sodium hydroxide, potassium hydroxide, sodium carbonate, and sodium hydrogen carbonate.

Examples of the antioxidant include ascorbic acid, sodium sulfite, butylhydroxyanisole, butylhydroxytoluene, propyl gallate, and tocopherol.

Examples of the thickener include alginic acid, polyethylene glycol, hydroxypropylmethyl cellulose, and sodium carboxymethyl cellulose.

The pH of the solution is not particularly limited as long as when the solution is injected into the target, the biofunctional substance exhibits a physiological activity and stably exists in the target, and has no adverse effect such as destruction of the target.

### Target

The target in the present embodiment may be, for example, one or more selected from the group consisting of a cell, a cell sheet, a cell cluster, a tissue, an organ (skin, body part, or the like), an organ system, an individual (living body), and the like. It may be one or more selected from the group consisting of a tissue, an organ (skin, body part, or the like), an organ system, an individual (living body), and the like. Any of an in vitro system, an in vivo system, and an ex vivo system may be adoptable. The cell cluster may be a cell cluster obtained by three-dimensional culture, and the organ (skin, body part, or the like) may be an organoid.

In a case of being injected into the target, the solution may be injected into a lower layer included therein. That is, for example, in a case in which an individual (living body) is targeted, the solution may be injected into a tissue included in the individual (living body), may be injected into a cell included in the individual (living body), or may be injected into both. In a case in which a tissue is targeted, the solution may be injected into a cell included in the tissue, may be injected into an extracellular matrix included in the tissue, or may be injected into both. In a case in which a cell is targeted, the solution may be injected into a cytoplasm of the cell, may be injected into a cell nucleus of the cell, or may be injected into both the cytoplasm and the cell nucleus of the cell.

In a case in which the target in the present embodiment is one or more selected from the group consisting of a cell, a cell sheet, a cell cluster, a tissue, an organ (skin, body part, or the like), and an organ system, the target may be one or more selected from the group consisting of a cell, a cell sheet, a cell cluster, a tissue, an organ (skin, body part, or the like), and an organ system in a state of existing in an individual (living body), or may be one or more selected from the group consisting of a cell, a cell sheet, a cell cluster, a tissue, an organ (skin, body part, or the like), and an organ system in a state of not existing in an individual (living body) (for example, a state of being extracted or isolated from the individual (living body) or a state of being prepared outside the individual (living body).

The target in the present embodiment may also be one or more selected from the group consisting of a cell, a cell sheet, a cell cluster, a tissue, an organ (skin, body part, or the like), and an organ system derived from a stem cell such as an induced pluripotent stem cell (iPS cell), and these may be in a state of existing in an individual (living body) or may be in a state of not existing in an individual (living body) (for example, a state of being extracted or isolated from the individual (living body) or a state of being prepared outside the individual (living body).

The individual (living body) is preferably a mammalian individual (living body). The mammalian individual is not particularly limited, but examples of the mammalian individual include humans and mammals other than the humans. Examples of the non-human mammal include mouse, rat, guinea pig, hamster, cattle, goat, sheep, pig, monkey, dog, and cat.

The body part may be one or more selected from the group consisting of fat, a skeletal muscle, a ligament, an aorta, a superior vena cava, a brain, an eyeball, an eardrum, an inner ear, a nerve, a trachea, a bronchus, a lung, an esophagus, a stomach, a liver, a kidney, an adrenal gland, a spleen, a gallbladder, a pancreas, a large intestine, a small intestine, a duodenum, a cecum, an appendix, a rectum, a greater omentum, a ureter, bone marrow, a lymph node, a lymphatic network, a thyroid gland, a sebaceous gland, a sweat gland, a salivary gland, a thymus, a mammary gland, a prostate, a fallopian tube, an ovary, a uterus, a cervix, a testicle, a vas deferens, and a seminal vesicle, as well as
a mucous membrane and a connective tissue associated with any one of these, as well as
a neoplasm occurring in any one of these.

These body parts are tissues that are more fragile than the skin and the like.

### Injector

As described above, the injector according to the present embodiment includes: an accommodation part configured to accommodate the solution; a nozzle part configured to communicate with the accommodation part, the nozzle part having an ejection port configured to eject the solution toward the target; and a pressurization part that ejects the solution from the ejection port toward the target by pressurizing the solution accommodated in the accommodation part when actuated, and injects the solution into the target by ejecting the solution from the ejection port of the nozzle part.

The injector according to the present embodiment may include a predetermined structure that guides the solution from an injector body to the target, for example, in a case in which the distance from the injector body to the target is long, such as a catheter, for example. Therefore, the injector according to the present embodiment may include or need not include such a predetermined structure.

In the injector according to the present embodiment, energy application by the pressurization part for pressurizing the solution can adopt a form of energy application by a known pressurization technique. The energy to be applied may be chemically generated energy such as combustion energy generated by an oxidation reaction of a low explosive or a high explosive, for example. Further, as another method, the energy for pressurizing may be generated electrically. As one example, energy caused by a piezoelectric element or an electromagnetic actuator driven by applied electric power may be employed. Alternatively, the energy for pressurization may be physically generated. Examples thereof include elastic energy by an elastic body and internal energy of a compressed object such as compressed gas. That is, the energy for pressurization may be any energy as long as the energy enables ejection of the solution in the injector. Further, the energy for pressurizing may be a composite-type energy obtained by appropriately combining the above-described combustion energy, energy generated by electric power, and internal energy such as the elastic energy.

From the above, for example, the pressurization part may pressurize by utilizing pressure generated by combustion of the explosive ignited by the ignition device, or may pressurize by utilizing pressure generated when the compressed gas is released. Further, the pressurization part may apply pressure by utilizing a biasing force of a compression spring, or may apply pressure by utilizing an electromagnetic force such as, for example, a linear electromagnetic actuator. The pressurization part preferably has at least a mode of utilizing pressure generated by combustion of the explosive ignited by the ignition device, and may further be utilized in combination with any of the other pressurizing modes described above.

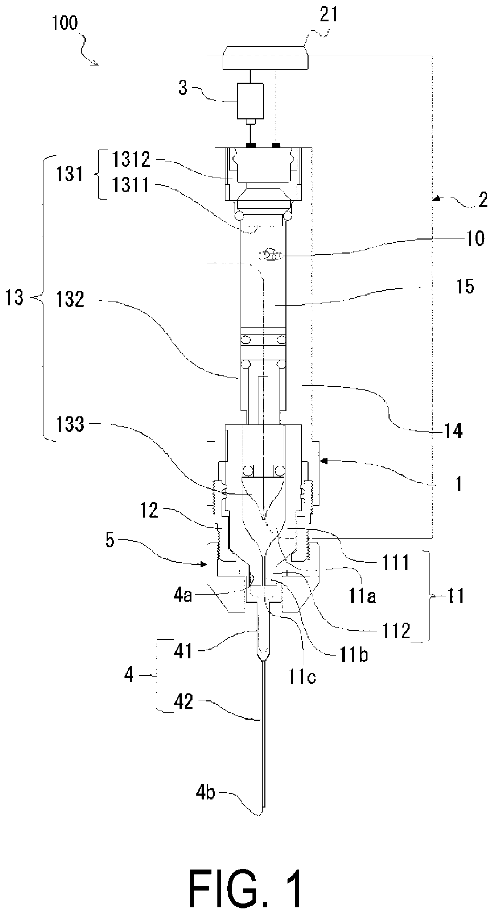

Hereinafter, an injector 100 illustrated in FIGS. 1 to 3 will be described as an example of an injector according to the present embodiment with reference to the drawings. Note that configurations of the following embodiment are provided as examples, and the technique of the present disclosure is not limited to the configurations of this embodiment. Note that, in the following description, the terms "tip end side" and "base end side" are used as terms indicating a relative positional relationship in a longitudinal direction of the injector 100. The "tip end side" refers to an outlet port 4b side illustrated in FIG. 1 and the like in the longitudinal direction of the injector 100, and the "base end side" refers to a side opposite to the outlet port 4b side in the longitudinal direction of the injector 100.

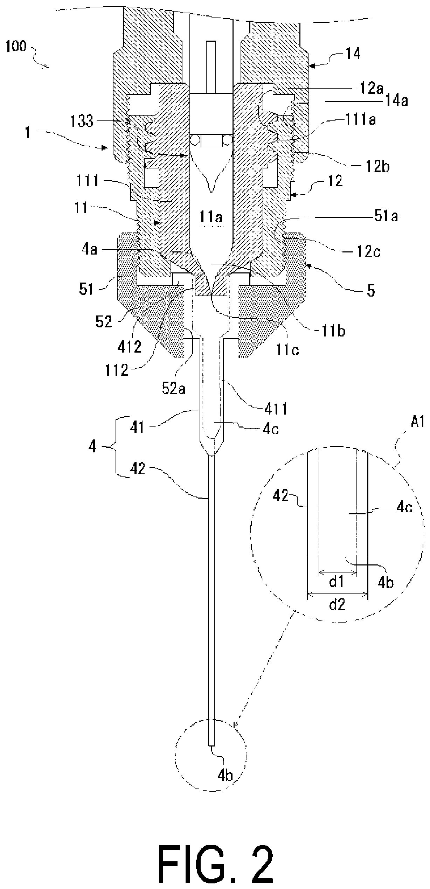

FIG. 1 is an overall view of the injector 100 according to the present embodiment. FIG. 1 illustrates a cross section along the longitudinal direction of the injector 100. Further, FIG. 2 is a cross-sectional view illustrating the vicinity of a tip end portion of the injector 100. As illustrated in FIG. 1, the injector 100 includes a jet injector 1 as an injector body, a housing 2 that detachably accommodates the jet injector 1, an injection needle 4 as an ejection part, and a fixing jig 5 for fixing the injection needle 4 to the jet injector 1.

### Jet Injector

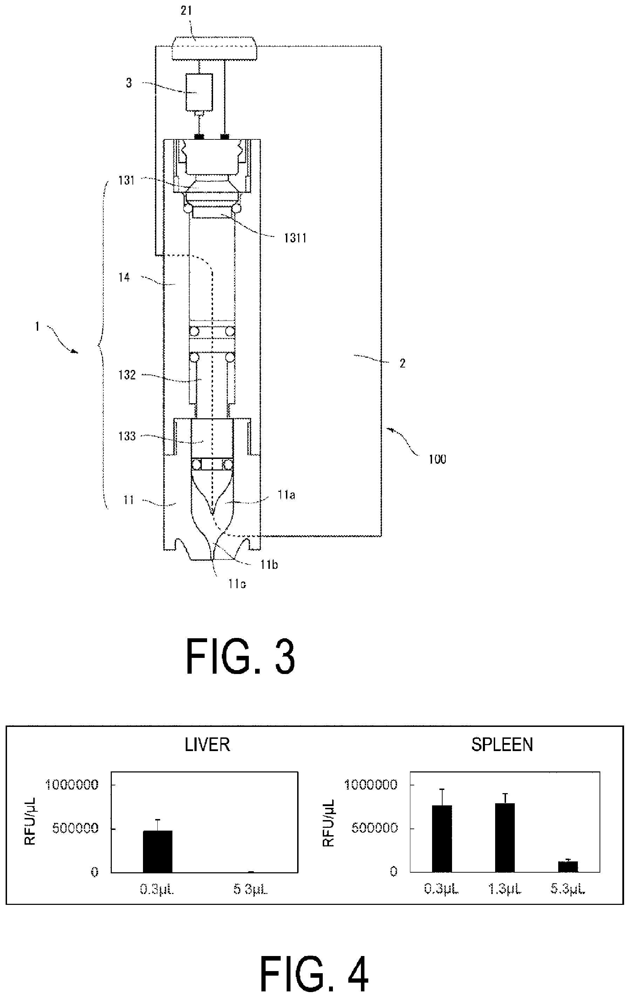

As illustrated in FIG. 1, the jet injector 1 includes a container 11, a container holder 12, an actuator 13, and a casing 14, which are integrally assembled to form an assembly. An ejection port denoted by reference sign 11c is formed at a tip end of the jet injector 1. The jet injector 1 pressurizes the solution containing the biofunctional substance (hereinafter, also simply referred to as a "solution") utilizing the combustion energy of an explosive, thereby ejecting the solution from the ejection port 11c. The jet injector 1 according to the present embodiment is configured as a needleless injector capable of injection by itself without using an injection needle. The needleless injector injects the solution from the injector body into the target without performing injection through a predetermined structure in a state in which the predetermined structure is inserted into the target. Specifically, the injector 100 illustrated in FIG. 3 is referred to as a needleless injector. However, the jet injector 1 need not be a needleless injector. In the following, as illustrated in FIG. 1, there is a case in which the injector 100 including the injection needle 4 and the fixing jig 5 is referred to as a needle injector.

### Casing

As illustrated in FIG. 1, the casing 14 is a tubular member accommodating the actuator 13. An ignition device 131 (described below) of the actuator 13 is fitted into the base end side of the casing 14, and the container holder 12 is fitted into the tip end side of the casing 14. As illustrated in FIG. 2, a threaded part 14a for coupling the casing 14 and the container holder 12 is formed on an inner circumferential surface of the tip end side of the casing 14. The container holder 12 and the actuator 13 are coupled via the casing 14.

### Container

As illustrated in FIG. 2, the container 11 includes a main body part 111 and a nozzle part 112 having a diameter smaller than that of the main body part 111, and is formed in a tubular shape. Further, inside the container 11 are formed an accommodating space 11a that is a space capable of accommodating the solution containing the biofunctional substance, and a flow path 11b that communicates with the accommodating space 11a and opens on the tip end side. More specifically, the accommodating space 11a is formed inside the main body part 111, and the flow path 11b is formed inside the nozzle part 112. The accommodating space 11a is an example of an accommodation part according to the present disclosure. An opening of the flow path 11b is formed in a tip end surface of the nozzle part 112, and the opening serves as the ejection port 11c of the jet injector 1. That is, the jet injector 1 ejects the solution from the ejection port 11c formed in the tip end surface of the nozzle part 112. As illustrated in FIG. 2, an inner diameter of the flow path 11b of the container 11 is smaller than an inner diameter of the accommodating space 11a. With such a configuration, the solution pressurized to a high pressure is ejected to the outside through the ejection port 11c of the flow path 11b. Further, a threaded part 111a for coupling the container 11 and the container holder 12 is formed on an outer circumferential surface of the main body part 111.

A material of the container 11 is not particularly limited. For example, the container 11 can be formed of a resin material. As the resin material forming the container 11, examples of materials that can be used include known materials such as nylon 6-12, polyarylate, polycarbonate, polybutylene terephthalate, polyphenylene sulfide, or liquid crystal polymer.

### Container Holder

As illustrated in FIG. 2, the container holder 12 is formed in a tubular shape and holds the container 11 fitted into the container holder 12. A threaded part 12a is formed on an inner circumferential surface of the base end side of the container holder 12. The threaded part 111a of the container 11 and the threaded part 12a of the container holder 12 are screwed together, thereby coupling the container 11 and the container holder 12. Further, a threaded part 12b is formed on an outer circumferential surface of the base end side of the container holder 12. The threaded part 12b of the container holder 12 and the threaded part 14a of the casing 14 are screwed together, thereby coupling the container holder 12 and the casing 14. Further, a threaded part 12c for coupling the container holder 12 and the fixing jig 5 is formed on the outer circumferential surface of the tip end side of the container holder 12.

A material of the container holder 12 is not particularly limited. For example, the container holder 12 can be formed of a metal material. Examples of the metal material forming the container holder 12 include stainless steel, copper, aluminum, iron, titanium, and a titanium alloy.

### Actuator

The actuator 13 illustrated in FIG. 1 is configured to pressurize the solution when actuated. The actuator 13 is an example of a "pressurization part" according to the present disclosure. As illustrated in FIG. 1, the actuator 13 includes the ignition device 131 and a piston 132 accommodated in the casing 14, and a plunger 133.

The ignition device 131 is disposed on the base end side of the casing 14, the plunger 133 is disposed on the tip end side of the casing 14, and the piston 132 is disposed adjacent to the plunger 133 and between the ignition device 131 and the plunger 133. A combustion chamber 15 is formed between the ignition device 131 and the piston 132 in an internal space of the casing 14.

### Ignition Device

The ignition device 131 includes an initiator 1311 and a holding member 1312. The initiator 1311 serves as a drive source of the actuator 13 and generates energy for pressurization and ejection of the solution by the injector 100. The initiator 1311 is configured as an electric igniter that releases a combustion product by burning an ignition agent accommodated in an interior thereof. The holding member 1312 is formed by injection molding of a resin. The injection molding can be performed by a known method. Further, for the holding member 1312, a resin material similar to that of the container 11 can be used. The ignition device 131 is configured as an igniter assembly in which the initiator 1311 is fixed to a base end portion of the casing 14 via the holding member 1312, and is fitted into the casing 14, closing the base end portion of the casing 14. Further, the ignition device 131 is disposed in the casing 14 with the initiator 1311 facing a base end surface (end surface on the base end side) of the piston 132, and thus the combustion energy of the ignition agent by the initiator 1311 and the combustion energy of a gas generating agent 10 described below are transmitted to the base end surface.

### Ignition Agent

Here, examples of the ignition agent used for the initiator 1311 include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and ferric oxide (AFO), and an explosive composed of a combination of a plurality of these explosives. These explosives exhibit characteristics that, although the explosives generate high-temperature and high-pressure plasma during combustion immediately after ignition, when the combustion product condenses at room temperature, the explosives do not contain gaseous components and accordingly the pressure generated decreases abruptly. Note that an explosive other than these may be used as the ignition agent as long as appropriate ejection of the solution can be performed.

### Gas Generating Agent

In the jet injector 1, to adjust the transition of the pressure applied to the solution via the piston 132, the gas generating agent 10 burned by the combustion product of the ignition agent released from the initiator 1311 to generate gas is disposed in the combustion chamber 15. That is, the jet injector 1 is configured to pressurize the solution by utilizing the combustion energy of the gas generating agent 10 in addition to the combustion energy of the ignition agent by the initiator 1311. The gas generating agent 10 is disposed at a location that can be exposed to the combustion product from the initiator 1311. Alternatively, the gas generating agent 10 may be disposed in the initiator 1311 as disclosed in WO 01/031282, JP 2003-25950 A, and the like. One example of the gas generating agent may be exemplified by a single base smokeless explosive (GG) formed of 98 mass% of nitrocellulose, 0.8 mass% of diphenylamine, and 1.2 mass% of potassium sulfate. Furthermore, various types of gas generating agents used in a gas generator for an air bag and a gas generator for a seat belt pretensioner can be used. A combustion completion time period of the gas generating agent can be changed by adjusting a dimension, a size, and a shape, particularly, a surface shape, of the gas generating agent at the time of being disposed in the combustion chamber 15. With this, the transition of the pressure applied to the solution is adjusted, and an ejection pressure can be transitioned in a desired manner. Note that the jet injector 1 need not include the gas generating agent 10 and may pressurize the solution by utilizing only the combustion energy of the ignition agent by the initiator 1311. In the present disclosure, the gas generating agent and the like to be used as necessary are also included in the pressurization part.

### Piston

The piston 132 is disposed on the tip end side of the casing 14, facilitating sliding inside the casing 14 by being pressurized by actuation of the initiator 1311. The piston 132 is formed of a metal, and an O-ring or the like may be disposed on a portion thereof to enhance sealing with a sliding surface (that is, inner circumferential surface of the casing 14) on which the piston 132 slides. Alternatively, the piston 132 may be formed of a resin and, in such a case, a metal may be used in combination for a portion requiring heat resistance and pressure resistance.

### Plunger

The plunger 133 is a member that receives the combustion energy of the ignition agent by the initiator 1311 and the combustion energy of the gas generating agent via the piston 132, thereby pressurizing the solution accommodated in the accommodating space 11a. The plunger 133 is accommodated in the casing 14 and disposed between the piston 132 and the nozzle part 112 of the container 11. The plunger 133 is formed in a rod shape, and a base end portion thereof is engaged with a tip end portion of the piston 132. Further, a tip end portion of the plunger 133 is inserted into the main body part 111 of the container 11, and the accommodating space 11a is defined by the plunger 133 and the main body part 111. The plunger 133 is slidable inside the main body part 111 and, with the sliding of the plunger 133, the solution accommodated in the accommodating space 11a is pressurized, passes through the flow path 11b, and is ejected from the ejection port 11c. Thus, the plunger 133 is formed of a material that smoothly slides against the main body part 111 of the container 11 and prevents the solution from leaking from the plunger 133 side.

Specific examples of the material of the plunger 133 include butyl rubber and silicone rubber. Further, for the purpose of securing and adjusting slidability between the plunger 133 and the main body part 111 of the container 11, an outer circumferential surface of plunger 133 and the inner circumferential surface of the main body part 111 may be subjected to coating or surface finishing with various substances.

Here, a contour of a tip end portion of the plunger 133 is formed in a shape substantially coinciding with a contour of a tip end portion of the accommodating space 11a. In this way, during ejection of the solution, when the plunger 133 slides and reaches a deepest position located furthest into the main body part 111, the accommodating space 11a formed between the plunger 133 and the nozzle part 112 can be reduced to the extent possible, and the solution can thus be inhibited from remaining in the accommodating space 11a and being wasted.

### Housing

The housing 2 illustrated in FIG. 1 is a member that accommodates the jet injector 1 and functions as a grip part that a user grasps to use the injector 100. As illustrated in FIG. 1, a battery 3 for supplying a drive current to the actuator 13 (more specifically, the initiator 1311) of the jet injector 1 is provided inside the housing 2. Further, as illustrated in FIG. 1, an outer surface of the housing 2 is provided with a plurality of switches 21 for operating the jet injector 1 to achieve ejection of the solution. Further, an inner surface of the housing 2 is provided with a socket (not illustrated) connected to the initiator 1311 of the jet injector 1. Power from the battery 3 to the jet injector 1 is supplied between an electrode on the housing 2 side and an electrode on the initiator 1311 side of the jet injector 1 via wiring by a press operation of the switch 21 by the user. Further, a control unit (not illustrated) such as a microcomputer is provided inside the housing 2. The control unit controls supply of an ignition current to the initiator 1311 of the jet injector 1 on the basis of a signal from each switch, thereby performing operation control of the jet injector 1.

Note that, as described above, in the present embodiment, the power for actuating the initiator 1311 is supplied from the battery built into the housing 2. However, instead of this mode, the power may be supplied from the outside via a power cable.

### Injection Needle

As illustrated in FIG. 1, the injection needle 4 is attached to the nozzle part 112 of the jet injector 1 and includes a base section 41 and a needle tube 42. The injection needle 4 is an example of an "ejection part" according to the present disclosure, and has a configuration in which a solution pressurized by the actuator 13 of the jet injector 1 flows in, and the solution that has flowed in can be ejected to the target.

The solution pressurized by the actuator 13 flows into the base section 41. As illustrated in FIG. 1, the base section 41 is formed in a tubular shape. More specifically, as illustrated in FIG. 2, the base section 41 includes a base body 411 having a tubular shape and a flange part 412 formed at a base end portion of the base body 411 and protruding outward in a radial direction. The nozzle part 112 of the jet injector 1 is press-fitted into an opening on the base end side of the base body 411. Therefore, the opening on the base end side of the base body 411 is configured as an inlet port 4a through which the solution containing the biofunctional substance can flow into the injection needle 4 from the accommodating space 11a of the container 11.

The base section 41 can be formed of, for example, a resin material. As the resin material forming the base section 41, a known synthetic resin such as polycarbonate, polypropylene, or polyethylene, for example, can be adopted. Further, the base section 41 may be made of a metal. Examples of the metal material forming the base section 41 include stainless steel, aluminum, an aluminum alloy, titanium, and a titanium alloy. The material of the base section 41 is not particularly limited, but in consideration of suppression of pressure loss of the solution flowing into the injection needle 4, a material having relatively high rigidity such as stainless steel is preferably used for the base section 41.

The needle tube 42 is inserted into the injection target and ejects the solution containing the biofunctional substance to the target. A base end portion of the needle tube 42 is connected to a tip end of the base section 41, and an internal space of the base section 41 and an internal space of the needle tube 42 communicate with each other. Therefore, the opening on the tip end side of the needle tube 42 is configured as an outlet port 4b that can eject, to the target, the solution containing the biofunctional substance flowing into the injection needle 4 from the inlet port 4a. A sharp needle tip for puncturing the skin is formed at a tip end portion of the needle tube 42 and thus the needle tube 42 can be inserted into the target.

Here, from the viewpoint of suppressing tissue damage of the target, the injection needle 4 (more particularly, the needle tube 42 to be inserted into the object) is preferably thin. As illustrated in an enlarged view A1 of FIG. 2, an inner diameter of the needle tube 42 is defined as d1, and an outer diameter of the needle tube 42 is defined as d2. At this time, the inner diameter d1 is preferably 1.5 mm or less, and more preferably 0.41 mm or less. The outer diameter d2 is preferably 1.7 mm or less, and more preferably 0.72 mm or less. However, the ranges of the inner diameter and the outer diameter of the needle tube 42 are not limited to the above. The length of the needle tube 42 can be appropriately set according to the target, and for example, a value selected from the range of 1 mm or more and 180 mm or less can be used.

A material of the needle tube 42 is not particularly limited, but examples thereof include stainless steel. Further, as a metal material other than stainless steel, aluminum, an aluminum alloy, titanium, a titanium alloy, or the like can be adopted. Further, the needle tube 42 may be formed of a resin material.

As described above, the internal space of the base section 41 and the internal space of the needle tube 42 communicate with each other, and thus, in the injection needle 4, a flow path 4c from the inlet port 4a to the outlet port 4b is formed by the internal space of the base section 41 and the internal space of the needle tube 42. The solution flowing into the injection needle 4 from the inlet port 4a flows through the flow path 4c to reach the outlet port 4b, and is ejected from the outlet port 4b to the target.

A sealant may be used between the nozzle part 112 of the container 11 and the base section 41 of the injection needle 4 to improve liquid-tightness. Examples of the sealant include an O-ring, packing, sealing tape, and a liquid sealant.

### Fixing Jig

The fixing jig 5 is a member for fixing the injection needle 4 to the nozzle part 112 of the jet injector 1. The fixing jig 5 includes a surrounding wall part 51 having a tubular shape and a lid wall part 52 that closes a tip end of the surrounding wall part 51. The surrounding wall part 51 is fitted into the container holder 12. Further, a threaded part 51a is formed on an inner circumferential surface of the surrounding wall part 51, and the container holder 12 and the fixing jig 5 are coupled by screwing the threaded part 12c of the container holder 12 and the threaded part 51a of the fixing jig 5 to each other. The lid wall part 52 is formed with a through hole 52a passing through the lid wall part 52 from the base end side to the tip end side. The base body 411 of the injection needle 4 is fitted into the through hole 52a. At this time, the lid wall part 52 presses the flange part 412 of the injection needle 4 from the tip end side, thereby preventing the injection needle 4 from falling off the nozzle part 112 of the jet injector 1. In this way, the injection needle 4 is fixed to the nozzle part 112 by the fixing jig 5.

A material of the fixing jig 5 is not particularly limited, but the fixing jig 5 can be formed of a metal material such as stainless steel, copper, aluminum, iron, titanium, or a titanium alloy, for example, as with the container holder 12. Further, for example, the fixing jig 5 may be formed of a resin material such as polycarbonate, polypropylene, or polyethylene.

### Operation of Injector

Injection of the solution into the target by the injector 100 illustrated in FIG. 1 is performed by operating the injector 100 in a state in which the needle tube 42 of the injection needle 4 is inserted into the target. As illustrated in FIG. 3, in a case in which the injector 100 is used as a needleless injector, the injector 100 is operated in a state in which the ejection port 11c is in contact with the target. The operation of the injector 100 will be described below. The solution containing the biofunctional substance may be accommodated in the accommodating space 11a of the container 11 from the beginning, or may be accommodated in the accommodating space 11a by sucking the solution from the outside of the injector 100 through the outlet port 4b of the injection needle 4 or the ejection port 11c. In a case in which the accommodation part and the ejection part are integrally molded, the solution containing the biofunctional substance is accommodated in the accommodating space 11a of the container 11 from the start, making it possible to reduce medical liquid leakage and dead volumes.

A gas may be accommodated in the accommodation part in addition to the solution containing the biofunctional substance. In this case, the solution is injected into the target while the gas dissolves in the solution together with pressurization. It is presumed that after being injected into the target, a part of the gas dissolved in the solution returns to the gas together with the pressure reduction, thereby generating an infinite number of fine bubbles, and a plurality of voids can be formed in the target by the shearing action of the bubbles. The void usually has a maximum diameter of about 10 µm to about 1000 µm, but a plurality of voids may communicate with each other to form larger voids. Formation of voids can be confirmed by using a stereomicroscope or the like.

The void can be utilized as a flow path or a reservoir for any liquid or gas, for example.

The gas that can be accommodated in the accommodation part is not particularly limited, but air is one example. The ratio of the volume of the gas to the total volume of the solution and the gas is not particularly limited, but is preferably 30% or more and more preferably more than 60%. It is preferably less than 100%, and more preferably 80% or less. That is, preferable ranges of the ratio of the volume of the gas include 30% or more and less than 100%, 30% or more and 80% or less, and more than 60% and 80% or less.

When the drive current is supplied to the actuator 13 of the jet injector 1 (more specifically, the initiator 1311) by operation of the switches 21 by the user and the actuator 13 is actuated, the combustion product of the ignition agent is released from the initiator 1311 to the combustion chamber 15. Thus, the gas generating agent 10 disposed in the combustion chamber 15 is burned by the combustion product of the ignition agent, generating gas in the combustion chamber 15. As described above, the base end surface of the piston 132 is exposed to the combustion chamber 15. Therefore, when the actuator 13 is actuated, the piston 132 receiving the combustion energy (pressure) of the ignition agent or the gas generating agent at the base end surface slides toward the tip end side of the casing 14. Thus, the plunger 133 is pushed toward the tip end side of the accommodating space 11a by the piston 132, and the solution in the accommodating space 11a is pressurized. As a result, the solution passes through the flow path 11b from the accommodating space 11a and is ejected from the ejection port 11c formed in the nozzle part 112. In a case of using the needleless injector illustrated in FIG. 3, this enables the solution to be injected into the target.

In a case of using the injection needle 4 and the fixing jig 5 as illustrated in FIGS. 1 and 2, the solution ejected from the ejection port 11c of the nozzle part 112 flows into the injection needle 4 through the inlet port 4a of the injection needle 4 attached to the nozzle part 112. The solution flows through the flow path 4c to be ejected from the outlet port 4b to the target. As described above, the operation of the injector 100 is completed.

The injection of the solution into the target by the injector in the present embodiment may be jet injection into the target.

Here, "jet injection" in the present disclosure refers to an injection characterized in that the solution is ejected from the ejection port toward the target, thereby forming a through hole penetrating a boundary between the inside and the outside of the target, and a high-pressure ultra-micro liquid current that can inject the solution into the target through the through hole occurs. For example, in a case in which the target is an individual (living body) of a mammal, jet injection refers to an injection characterized in that a high-pressure ultra-micro liquid current that can penetrate the outer skin or the like of the individual (living body) of the mammal occurs.

In the present embodiment, a value in which the kinetic energy of the solution to be ejected is divided by an area of the ejection port is 40 mJ/mm² or more and 1500 mJ/mm² or less. The value represents the pressure applied to the target at the time of ejection. The value in which the kinetic energy of the solution to be ejected is divided by the area of the ejection port is preferably 40 mJ/mm² or more and 1000 mJ/mm² or less, more preferably 40 mJ/mm² or more and 300 mJ/mm² or less, and particularly preferably 40 mJ/mm² or more and 100 mJ/mm² or less. The value in which the kinetic energy of the solution to be ejected is divided by the area of the ejection port is within the above range, whereby it is possible to prevent the ejected solution from penetrating the target while performing jet injection into the target.

The kinetic energy of the solution is expressed by 1/2 × m × v², where the mass of the solution is m (kg) and the ejection speed is v (m/s).

In the present disclosure, the ejection speed is a speed of the solution when ejected from the ejection port. The ejection speed can be calculated by capturing, with an image capturing device such as a high-speed camera at 20,000 fps, the manner in which a solution is ejected from the ejection port, for example, and dividing a distance by 50 µs, which is an image capturing interval, the distance traveled by the tip end of the ejected solution while two consecutive images are captured.

The ejection speed can be set within the above range by adjusting the ejection energy according to the shape and material of the nozzle part as well as the volume, viscosity, and the like of the solution.

In the present disclosure, the ejection port refers to an opening through which a solution is ejected from an integrally assembled injector. In the injector 100, when the injector 100 is used as a needleless injector, the ejection port is the ejection port 11c, and when the injector 100 is used as a needle injector, the ejection port is the outlet port 4b.

The area of the ejection port is preferably 0.001 mm² or more and 1.25 mm² or less, more preferably 0.001 mm² or more and 0.1 mm² or less, and particularly preferably 0.001 mm² or more and 0.02 mm² or less. When the area of the ejection port is within the above range, it is easy to perform jet injection to the target.

The injector according to the present embodiment can usually eject the solution at an ejection speed of more than 83.3 µL/s. The lower limit of the ejection speed is preferably 200 µL/s or more, more preferably 250 µL/s or more, still more preferably 300 µL/s or more, yet still more preferably 350 µL/s or more, and most preferably 400 µL/s or more. The upper limit of the ejection speed is preferably 5000 µL/s or less, more preferably 1000 µL/s or less, and still more preferably 700 µL/s or less. That is, preferable ranges of the ejection speed include more than 83.3 µL/s and 5000 µL/s or less, 200 µL/s or more and 1000 µL/s or less, 250 µL/s or more and 700 µL/s or less, 300 µL/s or more and 700 µL/s or less, and 350 µL/s or more and 700 µL/s or less.

When the ejection speed is within the above range, it is easy to perform jet injection into the target.

The lower limit of the ejection speed of the injector according to the present embodiment is preferably 40 m/s or more, and more preferably 45 m/s or more. The upper limit of the ejection speed is preferably 100 m/s or less, and more preferably 90 m/s or less. That is, preferable ranges of the ejection speed include 40 m/s or more and 100 m/s or less and 45 m/s or more and 90 m/s or less.

The fluid volume of the solution is preferably 0.3 µL or more and 4.0 µL or less. It is more preferably 0.3 µL or more and 1.5 µL or less, still more preferably 0.3 µL or more and 1.0 µL or less. When the fluid volume of the solution is within the above range, it is easy to prevent the ejected solution from penetrating the target.

The time required for injection of the solution into the target is not particularly limited as long as the solution of the injection amount can be ejected at the ejection speed, and examples thereof include 0.001 seconds or more, 0.005 seconds or more, or 0.01 seconds or more. Examples thereof include 100 seconds or less, 50 seconds or less, and 10 seconds or less. That is, examples thereof include 0.001 to 100 seconds, 0.005 to 50 seconds, and 0.01 to 10 seconds.

Use of the injector according to the present embodiment enables the solution containing the biofunctional substance to be injected into the target. That is, an embodiment of the present disclosure is a method for injecting a solution containing the biofunctional substance into the target using the injector.

The above method may include:
accommodating the solution in the accommodation part;
pressurizing the solution accommodated in the accommodation part; and
injecting the solution into the target.

Details of the injection target, the method for accommodating the solution in the accommodation part, the method for pressurizing the solution accommodated in the accommodation part, and the method for injecting the solution into the target can be referred to in the description of the injector.

### Examples

Hereinafter, the present disclosure will be specifically described with reference to Examples. However, the present disclosure is not limited to Examples as described below.

### Experiment 1: Investigation of Ejection Conditions

Actranza lab. (trade name) (for mouse intradermal administration, corresponding to the needleless injector illustrated in FIG. 3) equipped with a container rated at 20 µL was used for an ejection experiment. The Actranza lab was filled with 1% (w/v) malachite green (specific gravity: 1.0018) of the fluid volume of Table 1 and operated, thereby ejecting the solution into a transparent plastic tube with a graduation of 1 mm intervals. In the Actranza lab, ZPP was used as an ignition agent and GG (containing 98 mass% of nitrocellulose, 0.8 mass% of diphenylamine, and 1.2 mass% of potassium sulfate) was used as a gas generating agent, and the solution was administered using them. The manner of ejection was captured using a high-speed camera at 20,000 fps. The ejection speed was calculated by dividing the distance traveled by the tip end of the ejected solution between the first frame and the second frame in which the ejection of the solution was observed from the tip end of the nozzle by 50 µs, which is the capturing interval. Furthermore, kinetic energy was calculated with the specific gravity of the solution being 1.

Subsequently, the energy applied to the target was calculated by dividing the kinetic energy by 7.85 × 10⁻³ mm², which is the opening area of the ejection port of the Actranza lab.

Table 1 shows the fluid volume, the ejection speed, the kinetic energy, and the kinetic energy/opening area.

### [Table 1]

**Table 1**

| Fluid volume (µL) | Ejection speed (m/s) | Kinetic energy (mJ) | Kinetic energy/opening area (mJ/mm²) |
|---|---|---|---|
| 0.3 | 50.3 | 0.38 | 47.7 |
| 1.3 | 60.0 | 2.34 | 297.9 |
| 3.3 | 80.0 | 10.56 | 1344.5 |
| 5.3 | 80.0 | 16.96 | 2159.4 |
| 10.3 | 60.0 | 18.54 | 2360.6 |

### Experiment 2: Investigation of Tissue Penetration in Mouse Liver or Spleen

A triple anesthetic mixture (volume per mouse animal body weight, medetomidine: 0.3 mg/kg, midazolam: 4 mg/kg, butorphanol: 5 mg/kg) was intraperitoneally administered to an 8-week-old ICR mouse (Japan SLC, Inc., male). After confirming that there was no avoidance response when the lower limb was pinched with tweezers, the mouse was subjected to a laparotomy and the liver or spleen was exposed. To the exposed liver or spleen, 1% (w/v) malachite green was administered in the fluid volume described in Table 2 using the Actranza lab. The ignition agent and the gas generating agent were the same as those in Experiment 1. When diffusion of the pigment was confirmed immediately after administration, it was determined that the tissue was penetrated by injection. The results are shown in Table 2, where, among the three administrations, those in which tissue penetration was not observed at all were "A", those in which tissue penetration was not observed in at least one administration were "B", and those in which tissue penetration was observed in all three administrations were "C".

### [Table 2]

**Table 2**

| | | | | | | |
|---|---|---|---|---|---|---|
| Fluid volume (µL) | 0.3 | 1.3 | 3.3 | 5.3 | 10.3 | 20.3 |
| Liver | A | B | B | C | C | C |
| Spleen | A | A | B | C | C | C |

As shown in Table 2, when the value of the kinetic energy/opening area was 40 mJ/mm² or more and 1500 mJ/mm² or less, tissue penetration could be suppressed.

### Experiment 3: Gene Expression in Mouse Liver or Spleen

An accommodation part of the Actranza lab was filled with 0.3 µL, 1.3 µL, or 5.3 µL of a luciferase (Luc) expression plasmid pGL4 (hereinafter, sometimes referred to as pLuc). pGL4 was diluted in advance to 1 µg/µL using PBS. The ignition agent and the gas generating agent were the same as those in Experiment 1.

A triple anesthetic mixture was intraperitoneally administered to the mouse. After confirming that there was no avoidance response when the lower limb was pinched with tweezers, the mouse was subjected to a laparotomy and the liver or spleen was exposed. pLuc was administered by pressing the tip end of the Actranza lab against the body part of the target to operate. Thereafter, the abdomen was closed by ligating and suturing each of the muscle and the skin of the incision site.

After 24 hours, the mouse was subjected to cervical dislocation under anesthesia, death by exsanguination via the carotid artery was confirmed, and the target body part was harvested.

Luc activity was measured using a Luc-assay kit (Promega Corporation). The attached Passive Lysis buffer was added at 0.5 to 1 mL, and the tissue was pulverized with scissors for about 2 to 3 minutes. Subsequently, the specimens were frozen, thawed at room temperature, and centrifuged at 10,000 × g for 10 minutes at 16°C. Following the protocol, 20 µL of supernatant and 100 µL of substrate solution were mixed in a Lumi Tube (Kikkoman Corporation), and relative fluorescence units (RFUs) were promptly measured using a Lumitester C-110 (Kikkoman Corporation). The total RFU per tissue specimen was calculated by multiplying the RFU measurement value by a correction factor to obtain the RFU per total volume of added passive lysis buffer.

FIG. 4 shows values in which the total RFU is divided by the administered fluid volume.

As shown in FIG. 4, the administration condition evaluated as A in Experiment 2 had significantly higher gene expression efficiency than the condition evaluated as C.

### Reference Signs List

100 Injector
1 Jet injector
10 Gas generating agent
11 Container
11a Accommodating space
11b Flow path
11c Ejection port
111 Main body part
111a Threaded part
112 Nozzle part
12 Container holder
12a Threaded part
12b Threaded part
12c Threaded part
13 Actuator
131 Ignition device
1311 Initiator
1312 Holding member
132 Piston
133 Plunger
14 Casing
14a Threaded part
15 Combustion chamber
2 Housing
21 Switch
3 Battery
4 Injection needle
4a Inlet port
4b Outlet port
4c Flow path
41 Base section
411 Base body
412 Flange part
42 Needle tube
5 Fixing jig
51 Surrounding wall part
51a Threaded part
52 Lid wall part
52a Through hole

## Claims

1. An injector for injecting a solution containing a biofunctional substance into a target, the injector comprising:
an accommodation part configured to accommodate the solution;
a nozzle part configured to communicate with the accommodation part, the nozzle part having an ejection port for ejecting the solution toward the target; and
a pressurization part configured to eject the solution from the ejection port toward the target by pressurizing the solution accommodated in the accommodation part when actuated, wherein
a value in which a kinetic energy of the solution to be ejected is divided by an area of the ejection port is 40 mJ/mm² or more and 1500 mJ/mm² or less.

2. The injector according to claim 1, wherein a fluid volume of the solution is 0.3 µL or more and 4.0 µL or less.

3. The injector according to claim 1, wherein an area of the ejection port is 0.001 mm² or more and 1.25 mm² or less.

4. The injector according to claim 1, wherein an area of the ejection port is 0.001 mm² or more and 0.02 mm² or less.

5. The injector according to claim 1, wherein the biofunctional substance is one type or more selected from the group consisting of a nucleic acid, a peptide, a protein, a sugar, and a low-molecular-weight compound, as well as a complex thereof.

6. The injector according to claim 1, wherein
the target is one or more selected from the group consisting of fat, a skeletal muscle, a ligament, an aorta, a superior vena cava, a brain, an eyeball, an eardrum, an inner ear, a nerve, a trachea, a bronchus, a lung, an esophagus, a stomach, a liver, a kidney, an adrenal gland, a spleen, a gallbladder, a pancreas, a large intestine, a small intestine, a duodenum, a cecum, an appendix, a rectum, a greater omentum, a ureter, bone marrow, a lymph node, a lymphatic network, a thyroid gland, a sebaceous gland, a sweat gland, a salivary gland, a thymus, a mammary gland, a prostate, a fallopian tube, an ovary, a uterus, a cervix, a testicle, a vas deferens, and a seminal vesicle, as well as a mucous membrane and a connective tissue associated with any one of these,
as well as
a neoplasm occurring in any one of these.

7. A method for injecting the solution containing the biofunctional substance into the target using the injector according to any one of claims 1 to 6.
